# EUROPÄISCHE PATENTSCHRIFT

(11) **EP 1 261 388 B1**
(45) Veröffentlichungstag und Bekanntmachung des Hinweises auf die Patenterteilung: **24.05.2006**
(21) Anmeldenummer: 01903564.1
(22) Anmeldetag: 22.02.2001
(51) Int. Cl.: A61M 5/32

(54) **NADELSCHUTZVORRICHTUNG FÜR EIN INJEKTIONSGERÄT**
NEEDLE PROTECTING DEVICE FOR AN INJECTION UNIT
DISPOSITIF DE PROTECTION D'AIGUILLE POUR APPAREIL D'INJECTION

(30) Priorität: 01.03.2000 DE 10009816
(43) Veröffentlichungstag der Anmeldung: 04.12.2002
(73) Patentinhaber: TecPharma Licensing AG, 3401 Burgdorf (CH)
(72) Erfinder: HOMMANN, Edgar, CH-3257 Grossaffoltern (CH); HOSTETTLER, Peter, CH-3423 Ersigen (CH)
(86) Internationale Anmeldenummer: PCT/CH2001/000113
(87) Internationale Veröffentlichungsnummer: WO 2001/064271

(56) Entgegenhaltungen:
- US-A- 4 787 891
- US-A- 4 915 702
- US-A- 5 201 720
- US-A- 5 246 428
- US-A- 5 304 137
- US-A- 5 360 408
- US-A- 5 498 244
- US-A- 5 609 577

## Beschreibung

Die Erfindung betrifft eine Nadelschutzvorrichtung für ein Injektionsgerät, die in einer Schutzstellung eine Injektionsnadel des Injektionsgeräts umgibt und ein unbeabsichtigtes Freilegen der Nadel verhindert.

Aus US 4,915,702, ist eine Einwegspritze bekannt, die eine Nadelschutzhülse aufweist, die gegenüber einem Spritzengehäuse derart verschiebbar ist, dass eine Injektionsnadel aus einer Schutzposition, in der sie von der Nadelschutzhülse umgeben ist, in eine Nadelfreigabeposition gebracht werden kann. Die Nadelschutzhülse weist einen schwenkbaren Hebel auf, der in einem Lager an der Nadelschutzhülse schwenkbar gelagert ist. An dem Spritzengehäuse sind in einem Bereich nahe der Injektionsnadel erste Nuten und in einem Bereich entfernt von der Injektionsnadel zweite Nuten vorgesehen. Die Schwenkhebel können durch eine Öffnung in der Nadelschutzhülse mit einem hakenförmigen Armbereich durch die Nadelschutzhülse hindurch in die Nuten des Spritzengehäuses eingreifen. Auf diese Weise kann durch ein Eingreifen des Schwenkarmes in die nahe der Injektionsnadel gelegenen ersten Nuten die Nadelschutzhülse in einer Nadelschutzposition verriegelt werden. Greifen die Arme in die entfernt von der Injektionsnadel gelegenen zweiten Nuten ein, befindet sich die Nadelschutzhülse in einer Nadelfreigabeposition. Aus dieser Nadelfreigabeposition muss die Nadelschutzhülse durch aktives Betätigen der Arme, d.h. einem Lösen des Eingriffs in die Nuten, gelöst und von Hand in eine Schutzposition gebracht werden.

Aus der US-PS 5,609,577 ist ein Injektionsgerät mit einer Nadelschutzvorrichtung bekannt. Eine Injektionsnadel wird von einer Nadelschutzhülse umgeben. Die Nadelschutzhülse ist an einem Gehäuse des Injektionsgeräts axial verschiebbar gelagert. In der Schutzstellung wird die Nadelschutzhülse durch eine Sperre gegen ein unbeabsichtigtes Verschieben und Freigeben der Injektionsnadel gesichert. Nach einem Lösen der Sperre kann die Nadelschutzhülse in eine distale, hintere Stellung verschoben werden, in der die Injektionsnadel frei über die zurückgeschobene Nadelschutzhülse vorragt. Bei dem Zurückverschieben der Nadelschutzhülse wird eine Zugfeder der Nadelschutzvorrichtung gespannt. Wird die Nadelschutzhülse nach der Injektion entlastet, so schnapp die Nadelschutzhülse unter der Zugspannung der Zugfeder wieder in ihre Schutzstellung vor. Der Sperreingriff wird in der Schutzstellung automatisch wieder hergestellt.

Die Sperre wird durch einen Nocken und eine Führungsbahn für den Nocken gebildet. Die Führungsbahn besteht aus einem axial sich erstreckenden Abschnitt und einem sich rechtwinklig daran anschliessenden weiteren Abschnitt. In der Schutzstellung greift der Nocken in den abgewinkelten Abschnitt der Führungsbahn ein. Auf diese Weise wird die Nadelschutzhülse gegen ein Verschieben und Freilegen der Injektionsnadel gehindert. Zum Lösen der Sperre wird die Nadelschutzhülse relativ zu dem Basiskörper verdreht, bis der Nocken in den axial sich erstreckenden Abschnitt der Führungsbahn zu liegen kommt. In dieser Verdrehstellung kann die Nadelschutzhülse relativ zu dem Basiskörper in die Distalstellung verschoben werden. Die Zugfeder dient nicht nur dem Vorspannen der Nadelschutzhülse in die proximale, vordere Stellung. Gleichzeitig spannt sie die Nadelschutzhülse auch in die Verdrehposition, in der der Nocken in dem abgewinkelten Abschnitt der Führungsbahn auf Sperranschlag liegt. Die Nadelschutzhülse wird für die Injektion somit zunächst gegen die Kraft der Zugfeder verdreht und dann, ebenfalls gegen die Kraft der Zugfeder, in die Distalstellung verschoben.

Bei der bekannten Nadelschutzvorrichtung ist die Zugfeder ein wesentliches Element der Sperre. Durch die Verwendung einer Feder für die Sicherung der Nadelschutzhülse in der Schutzstellung ist eine aufwendige Konstruktion für den Sperrmechanismus erforderlich. Ferner ist das ordnungsgemäße Funktionieren des Sperrmechanismus wesentlich von der Feder abhängig. So kann die Feder beispielsweise brechen oder verhaken. Im Falle einer Fehlfunktion der Feder kann eine sichere Sperre der Nadelschutzhülse in der Schutzstellung nicht garantiert werden.

Es ist eine Aufgabe der Erfindung, eine Nadelschutzvorrichtung für ein Injektionsgerät zu schaffen, die in einer Schutzstellung eine Nadelschutzhülse gegen ein Verschieben sicher sperrt und störunanfällig ist. Vorzugsweise soll ein Sperrmechanismus eine möglichst geringe Anzahl von bewegbaren Teilen erfordern und einfach aufgebaut sein.

Eine Nadelschutzvorrichtung, wie die Erfindung sie betrifft, weist einen hülsenförmigen Basiskörper und eine Nadelschutzhülse auf. Eine Injektionsnadel des Injektionsgeräts steht über ein proximales, vorderes Ende des Basiskörpers hinaus. Die Nadelschutzhülse ist axial verschiebbar an dem Basiskörper gelagert. In einer proximalen Schutzstellung umgibt die Nadelschutzhülse die Injektionsnadel. Nach einer Axialverschiebung in eine hintere Stellung, im folgenden Distalstellung genannt, gibt die Nadelschutzhülse die Injektionsnadel frei. In der Schutzstellung wird die Nadelschutzhülse gegen ein Zurückschieben relativ zu dem Basiskörper und zu der Injektionsnadel gesperrt. In der Schutzstellung der Nadelschutzhülse besteht zwischen einem ersten Sperrmittel und einem zweiten Sperrmittel ein Sperreingriff, indem die beiden Sperrmittel axial gegeneinander stoßen. Eines der Sperrmittel ist verschiebesicher mit der Nadelschutzhülse verbunden, und das andere Sperrmittel ist verschiebsicher mit dem Basiskörper verbunden. Der Sperreingriff verhindert daher ein Verschieben der Nadelschutzhülse aus der Schutzstellung in die Distalstellung. Schließlich weist die Nadelschutzvorrichtung ein Entsicherungsmittel auf zum Lösen des Sperreingriffs zwischen dem ersten Sperrmittel und dem zweiten Sperrmittel.

Die Angaben "proximal" und "distal" sind auf die Spitze der Injektionsnadel bezogen.

Nach der Erfindung wird das erste Sperrmittel durch einen elastisch biegbaren Schnapper gebildet, der mit der Nadelschutzhülse oder dem Basiskörper steif verbunden ist. In der Schutzstellung der Nadelschutzhülse liegt der Schnapper in axialer Richtung auf Anschlag gegen das zweite Sperrmittel, das einen Schnapperanschlag bildet. Der Sperreingriff der Sperrmittel wird durch Biegen des Schnappers gelöst. Der Schnapper wird aus dem Sperreingriff mit dem Schnapperanschlag abgebogen. Durch das Biegen wird der Schnapper elastisch gespannt, d.h. es wird in dem Schnapper eine Rückstellkraft erzeugt. Der Schnapper ist in seiner Sperrstellung, die er in der Schutzstellung der Nadelschutzhülse einnimmt, vorzugsweise vollkommen entspannt.

Die erfindungsgemäße Nadelschutzvorrichtung benötigt für die sichere Sperre lediglich den elastisch biegbaren Schnapper und den Sperranschlag. Ein drittes Element, beispielsweise in Form der aus dem Stand der Technik bekannten Zugfeder ist nicht erforderlich. Die Anzahl, der für das Herstellen der Sperre benötigten Teile, ist reduziert. Der erfindungsgemäße Schnapper vereinigt in sich die Sperrfunktion des bekannten Nockens und die Rückhalte- bzw. Sicherungsfunktion der bekannten Feder.

Das zweite Sperrmittel ist bevorzugt an einer Mantelfläche des Basiskörpers oder der Nadelschutzhülse gebildet. Besonders bevorzugt ist das Sperrmittel eine Wandung, die in einer radialen Richtung oder exakt radial aus der Mantelfläche hervor- oder in die Mantelfläche hineinragt. Die Mantelfläche kann eine Innenmantelfläche oder eine Außenmantelfläche des Basiskörpers oder eine Innenmantelfläche oder eine Außenmantelfläche der Nadelschutzhülse sein. In einem bevorzugten Ausführungsbeispiel ist die das zweite Sperrmittel bildende Wandung an einer Innenmantelfläche des Basiskörpers gebildet. Das zweite Sperrmittel kann durch eine einzige Wandung oder auch mehrere Wandungen, insbesondere wie vorstehend beschrieben, gebildet werden.

In bevorzugter Ausführung ist das Entsicherungsmittel an dem Basiskörper relativ zu der Nadelschutzhülse bewegbar, besonders bevorzugt verschiebbar, gelagert. Eine Verschiebelagerung ist so ausgebildet, dass das Entsicherungsmittel bei einem Verschieben an dem Schnapper entlang gleitet und dabei in solch eine Richtung auf den Schnapper drückt, dass der Schnapper aus dem Sperreingriff gebogen wird. Der Druck auf den Schnapper wirkt vorzugsweise quer zur Richtung der Verschiebung des Entsicherungsmittels. Das Entsicherungsmittel kann aber beispielsweise auch in der Art eines Druckknopfs ausgebildet sein, der in Richtung seiner eigenen Verschiebung relativ zum Basiskörper gegen den Schnapper drückt. Obgleich das Entsicherungsmittel selbst auch den Schnapperanschlag bilden kann und in diesem Fall beim Lösen selbst aus dem Sperreingriff bewegt wird, ist vorzugsweise ein gesonderter Schnapperanschlag vorgesehen, der ständig axial verschiebsicher mit der Nadelschutzhülse oder dem Basiskörper verbunden ist. Der Schnapperanschlag liegt vorzugsweise neben dem Verschiebeweg des Entsicherungsmittels. Besonders bevorzugt handelt sich um einen mit dem Basiskörper vollkommenen starr verbundenen Anschlag.

Eine Nadelschutzvorrichtung mit einem relativ zur Nadelschutzhülse bewegbar angeordneten Entsicherungsmittel weist als Vorteil auf, dass die Nadelschutzhülse nicht selbst zum Lösen des Sperreingriffs der Sperrmittel betätigt werden muss. Ein Verwender muss daher nicht noch dann auf das Lösen des Sperreingriffs achten, wenn die Nadelschutzhülse bereits für die Injektion gegen die Haut gedrückt wird, wie dies bei Verwendung von beispielsweise der Nadelschutzhülse der US-PS 5,609,577 der Fall ist. Das Lösen des Sperreingriffs und das Zurückschieben der Nadelschutzhülse können durch das bewegbar angeordnete Entsicherungsmittel voneinander getrennt werden. Ferner kann der Sperreingriff beliebig oft gelöst und wieder hergestellt werden, während die Nadelschutzhülse in der Schutzstellung verbleibt. Es ist nicht erforderlich, dass nach einem Lösen des Sperreingriffs eine Injektion durchgeführt wird, um wieder die gesperrte Schutzstellung zu erhalten.

Vorzugsweise ist das Entsicherungsmittel in der Schutzstellung der Nadelschutzhülse so gesichert, dass es nicht von der Nadelschutzhülse mitgenommen wird, wenn die Nadelschutzhülse sich in ihre Schutzstellung bewegt. Vorteilhafterweise ist das Entsicherungsmittel dadurch auch gegen eine unbeabsichtigte Bewegung, beispielsweise aufgrund von Vibrationen, gesichert. Die Sicherung des Entsicherungsmittels erfolgt bevorzugt dadurch, dass in der Schutzstellung der Nadelschutzhülse zwischen dem Entsicherungsmittel und dem Basiskörper ein Rasteingriff besteht. Dementsprechend ist der Basiskörper mit einem ersten Rasteingriffsmittel und das Entsicherungsmittel mit einem zweiten Rasteingriffsmittel versehen. Der Rasteingriff ist lösbar.

Die Nadelschutzvorrichtung ist besonders bevorzugt so ausgebildet, dass die Nadelschutzhülse nach einer Injektion wieder in die Schutzstellung vorschiebt und in der Schutzstellung der erfindungsgemäße Sperreingriff des Schnappers und des Schnapperanschlags selbsttätig hergestellt wird. Die Nadelschutzvorrichtung erlaubt wiederholte Injektionen, wobei die Nadelschutzhülse durch den Sperrmechanismus stets wieder automatisch in ihrer Schutzstellung gegen ein unbeabsichtigtes Zurückschieben gesperrt wird. Vorzugsweise nimmt die Nadelschutzhülse bei ihrem Zurückschieben in die Distalstellung das Entsicherungsmittel bis in eine Stellung mit, in der das Entsicherungsmittel mit dem Basiskörper verrastet. Durch die bevorzugt lösbare Ausbildung dieses Rasteingriffs ist eine mehrfache Betätigung der Nadelschutzvorrichtung möglich. Grundsätzlich wäre es jedoch auch möglich, den Rasteingriff des Entsicherungsmittels unlösbar zu gestalten. Bei einem unlösbaren Rasteingriff wäre eine Nadelschutzvorrichtung mit einem selbsttätig in den Sperranschlag vorschnappenden Schnapper nur für eine Injektion verwendbar, wenn sichergestellt ist, dass der Sperreingriff des Schnappers nicht anderweitig als mit dem Entsicherungsmittel gelöst werden kann. Die Mitnahme des Entsicherungsmittels folgt bevorzugt dadurch, dass die Nadelschutzhülse bei ihrem Zurückschieben lose gegen das Entsicherungsmittel drückt.

Der Schnapper ist vorzugsweise als axial sich erstreckender Schnapperfinger mit einer stimseitigen Anschlagfläche ausgebildet. Vorzugsweise ist er einstückig mit dem Basiskörper oder, was besonders bevorzugt wird, einstückig mit der Nadelschutzhülse ausgebildet. Obgleich weniger bevorzugt, wäre es auch möglich, dass der Schnapper in einer radialen Richtung von dem Basiskörper oder der Nadelschutzhülse abragt.

Der Sperrmechanismus der Nadelschutzvorrichtung kann mehrere Schnapper zur Bildung mehrerer Sperreingriffe mit entsprechenden Sperranschlägen aufweisen. Mehrere Entsicherungsmittel, nämlich je wenigstens eines für jeden Schnapper, können auf einem gemeinsamen Träger relativ zueinander nicht verschiebbar angeordnet sein, so dass bei einer entsprechenden Betätigung des Trägers alle Sperreingriffe gleichzeitig gelöst werden. Die mehreren Entsicherungsmittel können jedoch auch unabhängig voneinander gelagert und betätigbar sein, um beispielsweise ein unbeabsichtigtes Lösen der Sperreingriffe noch sicherer zu verhindern.

In einem bevorzugten Ausführungsbeispiel wird der Sperrmechanismus von einem Hülsenteil des Basiskörpers umgeben, so dass von außen ein Zugriff nur auf das Entsicherungsmittel möglich ist. Das betreffende Hülsenteil des Basiskörpers erfüllt die Funktion einer Deckhülse. Die Nadelschutzhülse wird bei der Verschiebung in die Distalstellung in die Deckhülse geschoben, kann allerdings in der Distalstellung noch ein Stück weit über die Deckhülse hinaus vorragen.

In bevorzugter Ausführung ragt von dem Entsicherungsmittel ein Nocken ab und durch eine Durchbrechung der Deckhülse hindurch, und es ist das Entsicherungsmittel entlang der Deckhülse verschiebbar. Vorzugsweise ist der Nocken an dem Außenmantel der Deckhülse abgestützt, wodurch verhindert wird, dass durch das Betätigen ein radial einwärts gerichteter Druck auf das Entsicherungsmittel ausgeübt wird. Die bei der Betätigung des Entsicherungsmittels wirkenden Kräfte werden somit durch die Deckhülse abgefangen.

Der Basiskörper kann einteilig oder mehrteilig ausgebildet sein. Bei Ausbildung als mehrteiliger Basiskörper sind die mehreren Teil axial verschiebesicher miteinander verbunden. Der Basiskörper kann durch das Gehäuse des Injektionsgeräts gebildet werden, wie dies bei der Nadelschutzvorrichtung der US-PS 5,609,577 der Fall ist. In einer ebenfalls bevorzugten Ausführung ist die Nadelschutzvorrichtung als eigenständiges Gerät ausgebildet, dessen Basiskörper an ein Gehäuse eines Injektionsgeräts angepasst ist, derart, dass der Basiskörper mit dem Gehäuse des Injektionsgeräts verschiebesicher verbindbar ist. Besonders bevorzugt weist solch eine eigenständig handelbare Nadelschutzvorrichtung einen Basiskörper auf, der einfach auf das Gehäuse des Injektionsgeräts geschoben und an dem Gehäuse verschiebesicher fixiert wird. Besonders vorteilhaft ist die Ausbildung einer Aufsteckhülse, die auf ein bestehendes Injektionsgerät ganz einfach bis gegen einen Anschlag aufgesteckt wird und dann form- und reibschlüssig an dem Gehäuse des Injektionsgeräts fixiert ist. Die eigenständige Nadelschutzvorrichtung kann bei entsprechender Anpassung der Form ihres Basiskörpers vorteilhaft zur Nachrüstung von bestehenden Injektionsgeräten dienen, die eine Injektion auch ohne Nadelschutzvorrichtung erlauben.

Die Gegenstände der abhängigen Ansprüche bilden in Kombination bereits nur mit den Merkmalen a) bis f) von Anspruch 1 vorteilhafte Ausführungen einer Nadelschutzvorrichtung. Die Anmelderin behält es sich vor, auf solche Kombinationen einen eigenen Schutz zu richten.

Ein bevorzugtes Ausführungsbeispiel der Erfindung wird nachfolgend anhand von Figuren erläutert. Dabei werden weitere Merkmale und Vorteile offenbart. Die anhand des Ausführungsbeispiels offenbarten Merkmale bilden je einzeln und in Kombination die beanspruchte Erfindung und auch die genannten weiteren Erfindungen weiter. Es zeigen:
- Figur 1: eine Nadelschutzvorrichtung,
- Figur 2: einen Längsschnitt der Komponenten der Nadelschutzvorrichtung der Figur 1,
- Figur 3: eine perspektivische Darstellung der Komponenten der Figur 2,
- Figur 4: die Deckhülse der Nadelschutzvorrichtung nach den Figuren 1 bis 3,
- Figur 5: einen Längsschnitt der Nadelschutzvorrichtung nach den Figuren 1 bis 4 im montierten Zustand und
- Figur 6: ein Detail der Figur 5.

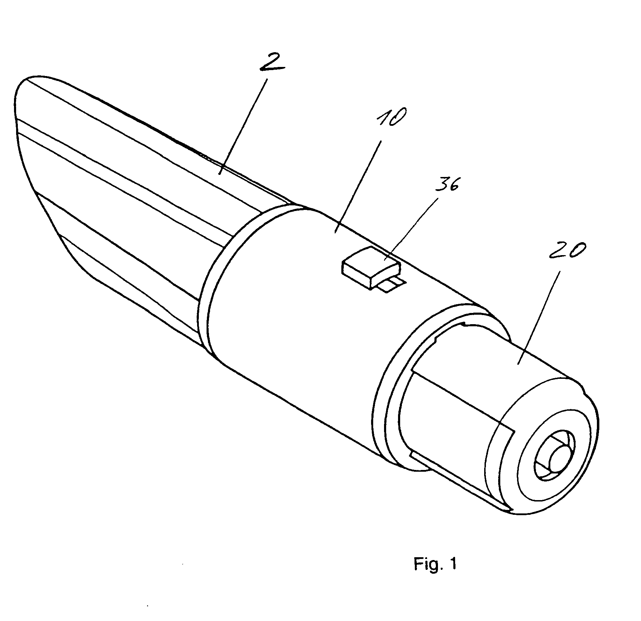

Figur 1 zeigt eine Nadelschutzvorrichtung im montierten Zustand. Zu erkennen sind eine Aufsteckhülse 2, eine Deckhülse 10 und eine Nadelschutzhülse 20 in koaxialer Anordnung. Die Deckhülse 10 ist auf die Aufsteckhülse 2 aufgeschnappt. Die Schnappverbindung ist praktisch nicht wieder lösbar. Im montierten Zustand können die Aufsteckhülse 2 und die Deckhülse 10 als ein einziger Basiskörper 2, 10 betrachtet werden. Insbesondere sind zwischen der Aufsteckhülse 2 und der Deckhülse 10 keine Verschiebebewegungen möglich. Die Nadelschutzvorrichtung ist auf ein Injektionsgerät zur Verabreichung eines Produkts, insbesondere eines Medikamentenfluids, wie beispielsweise Insulin, aufsteckbar. Hierfür ist die Aufsteckhülse 2 in Anpassung an das betreffende Injektionsgerät geformt.

Die Nadelschutzhülse ist in axialer Richtung innerhalb der Deckhülse 10 hin- und her verschiebbar. In Figur 1 nimmt sie eine Schutzstellung ein. In der Schutzstellung ist die Nadelschutzhülse 20 bis gegen einen innerhalb der Deckhülse 10 gebildeten Anschlag ausgefahren und umgibt eine Injektionsnadel des Injektionsgeräts bis über eine Nadelspitze hinaus. In der Schutzstellung schützt die Nadelschutzhülse 20 den Verwender vor Verletzungen durch die Injektionsnadel. Gleichzeitig wird die Injektionsnadel vor Beschädigungen geschützt. Ferner bildet die Nadelschutzhülse 20 in der Schutzstellung vorzugsweise einen Sichtschutz, so dass der Verwender die Injektionsnadel nicht sehen kann. Hierdurch wird insbesondere die zu beobachtende Hemmschwelle bei Verwendern, die sich das Produkt selbst injizieren, herabgesetzt.

Die Nadelschutzhülse 20 ist in der Schutzstellung gegen eine Verschiebung in die Deckhülse 10 hinein gesperrt. Durch die Sperrung wird ein unbeabsichtigtes Zurückschieben der Nadelschutzhülse 20 und Freilegen der Injektionsnadel verhindert. Unmittelbar vor einer Injektion muss der Verwender die Sperrung der Nadelschutzhülse 20 lösen, d.h. er muss die Nadelschutzvorrichtung entsichem. Dies geschieht durch eine Axialverschiebung eines Betätigungselements 36. Nach dem Lösen der Sperre kann die Nadelschutzhülse 20 gegen eine elastische Rückstellkraft relativ zu der Aufsteckhülse 2 und der Deckhülse 10 bis in eine Distalposition zurückgeschoben werden. In der Distalposition der Nadelschutzhülse 20 liegt die Injektionsnadel frei. Das Zurückschieben der Nadelschutzhülse erfolgt im Zuge des Einstechens bei der Injektion. Wird die Injektionsnadel nach der Injektion wieder aus dem Gewebe herausgezogen, wird die Nadelschutzhülse 20 durch die elastische Rückstellkraft wieder vorgeschoben. Sobald die Nadelschutzhülse 20 ihre Schutzstellung wieder erreicht hat, wird sie automatisch wieder gesperrt. Die Sperrung kann wiederholt gelöst werden.

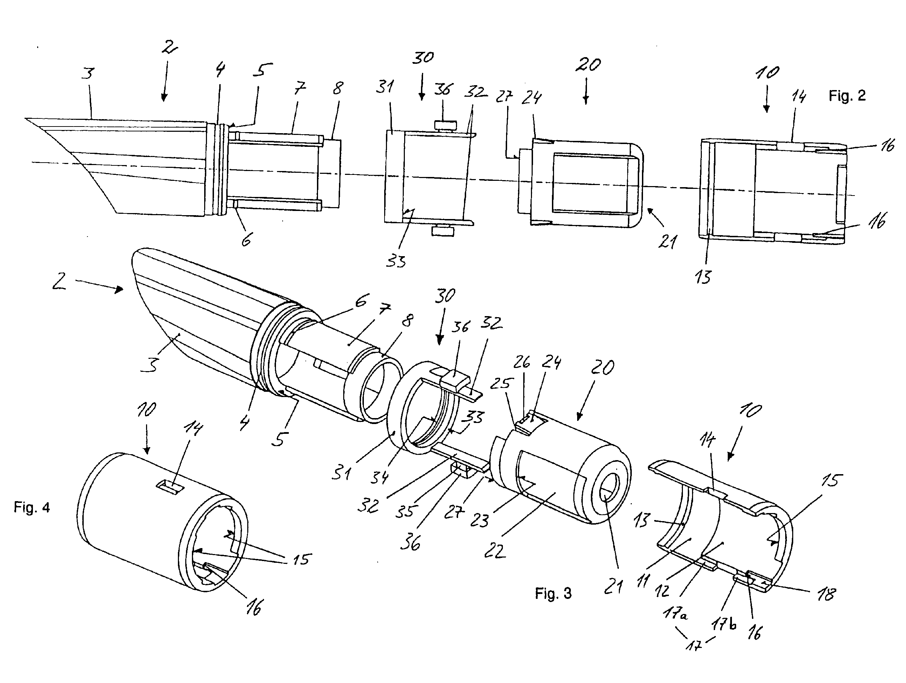

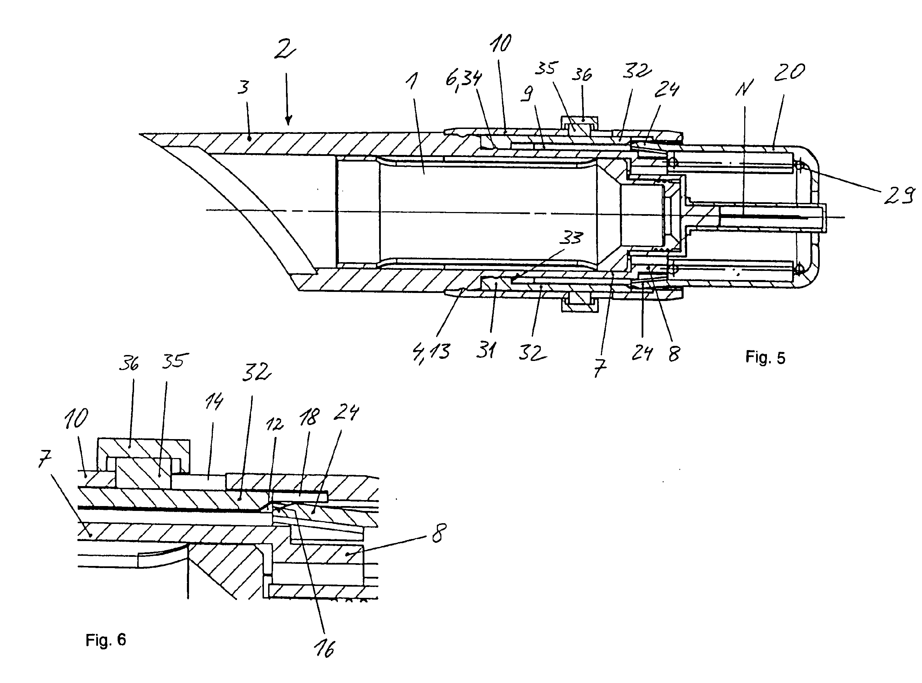

In den Figuren 2, 3 und 4 sind die Komponenten der Nadelschutzvorrichtung einzeln vor der Montage dargestellt. Die Nadelschutzvorrichtung wird durch fünf Komponenten gebildet, nämlich die Aufsteckhülse 2, die Deckhülse 10, die Nadelschutzhülse 20, ein Entsicherungsmittel 30 und ein Rückstellelement in Form einer Druckfeder 29, die allerdings nur in Figur 5 gezeigt ist. Im Ausführungsbeispiel muss allerdings nach der Montage dieser fünf Komponenten noch das Betätigungselement 36 mit dem Entsicherungsmittel 30 verbunden werden. Die vier Komponenten 2, 10, 20 und 30 und auch das Betätigungselement 36 sind je einstückig als Kunststoffspritzteile ausgeführt.

Die Aufsteckhülse 2 weist einen distalen, vollen Hülsenbereich 3 und einen proximalen Hülsenbereich mit zwei Hülsensegmenten 7 und einem Ring 8 auf. Die beiden Hülsensegmente 7 ragen um 180° versetzt von einer proximalen Stirnfläche des distalen Hülsenbereichs 3 ab. Durch den Ring 8 werden die beiden Hülsensegmente 7 an ihren proximalen Enden voneinander distanziert und so der proximale Hülsenbereich ausgesteift. In einem Übergangsbereich zwischen dem distalen Hülsenbereich 3 und den Hülsensegmenten 7 ist umlaufend eine Vertiefung bzw. Rille 4 ausgebildet. Schließlich weisen die beiden Hülsensegmente 7 nahe ihrer distalen Enden an ihren äußeren Mantelflächen je eine tangential sich erstreckende Verdickung bzw. einen Wulst 6 auf.

Die Deckhülse 10 weist eine Mantelfläche auf, die lediglich von zwei sich gegenüberliegenden Durchbrechungen 14 durchbrochen wird. In der Nähe des distalen Endes der Deckhülse 10 läuft an der Mantelinnenfläche eine Verdickung bzw. ein Wulst 13 um. Der Wulst 13 kommt im montierten Zustand in der Rille 4 der Aufsteckhülse 2 zu liegen. An dem proximalen Ende der Deckhülse 10 ragen zwei Schultersegmente 15 radial einwärts. Die beiden Schultersegmente 15 dienen als vordere Anschläge und als Geradführungen für die Nadelschutzhülse 20.

An der Innenmantelfläche der Deckhülse 10 sind in symmetrischer Anordnung und um 180° zueinander versetzt zwei Anschläge 16 ausgebildet. Die Anschläge 16 werden je durch ein Paar von Flächen gebildet, die dem proximalen Ende der Deckhülse 10 zugewandt sind und radial zur Innenmantelfläche der Deckhülse 10 weisen. Die Anschläge 16 sind in axialer Richtung gesehen zwischen den Durchbrechungen 14 und den Schultersegmenten 15 angeordnet. Im Innenmantel der Deckhülse 10 sind ferner zwei axial sich erstreckende, gerade Kanäle 17 ausgebildet. Je eine der Durchbrechungen 14 erstreckt sich durch einen der Kanäle 17 hindurch und unterteilt den jeweiligen Kanal in einen distalen Kanalabschnitt 17a und einen proximalen Kanalabschnitt 17b. Die Anschläge 16 grenzen unmittelbar an den proximalen Kanalabschnitt 17b des jeweiligen Kanals 17 und bilden so je eine Kante der Kanalberandung. Ein distaler Innenmantelbereich 11 der Deckhülse 10 ist gegenüber einem proximalen Innenmantelbereich 12 erweitert. Der Übergang wird durch eine umlaufende, durch die Kanäle 17 unterbrochene Schulter gebildet.

Die Nadelschutzhülse 20 weist die Form einer Kappe auf, indem sie an ihrem proximalen Ende einen Boden ausbildet, der lediglich von einer zentralen Öffnung 21 für die Injektionsnadel durchbrochen wird. Von einer distalen Stirnfläche der Nadelschutzhülse 20 ragen in symmetrischer Anordnung und um 180° versetzt zwei Schnapper 24 axial frei ab. Die Schnapper 24 ragen je ein kleines Stück radial über eine Außenmantelfläche der Nadelschutzhülse 20 hinaus. Die Schnapper 24 sind je in einem offenen Ausschnitt 25 der Mantelfläche der Nadelschutzhülse 20 ausgebildet. Die Schnapper 24 sind je plättchenförmig, im Ausführungsbeispiel sind es Rechteckplättchen. An ihren hinteren Enden sind die Schnapper 24 an ihren nach außen weisenden Oberseiten in einem Mittenbereich 26 verjüngt, vorzugsweise angeschrägt. Die Schnapper 24 federn in radialer Richtung um ihre Fußbereiche.

Schließlich weist die Nadelschutzhülse 20 an ihrer Außenmantelfläche einen vertieften Flächenbereich 22 auf, der zum proximalen Ende der Nadelschutzhülse 20 hin ausläuft und an seinem distalen Ende eine Schulter 23 zum Rest der Außenmantelfläche bildet. Ein weiterer vertiefter Flächenbereich 22 ist in gleicher Weise an der diametral gegenüberliegenden Seite der Nadelschutzhülse 20 ausgebildet.

Das Entsicherungsmittel 30 wird durch einen Ring 31 und zwei symmetrisch um 180° versetzt zueinander angeordnete, von dem Ring 31 axial abragende Zungen 32 gebildet. An der Innenmantelfläche des Rings 31 läuft eine Vertiefung bzw. Rille 34 um. Von den nach außen weisenden Oberflächen der Zungen 32 ragt je ein Nocken 35 nach außen ab. Auf jedem der Nocken 35 ist ein Betätigungselement 36 befestigt. Allerdings werden die Betätigungselemente 36 im Ausführungsbeispiel erst nach der Montage der vier Komponenten 2, 10, 20 und 30 auf dem Nocken 35 befestigt.

Bei der Montage wird zunächst die Nadelschutzhülse 20 in die Deckhülse 10 hineingeschoben. Die beiden Schultersegmente 15 bilden mit den Vertiefungen 22 eine Geradführung für die Nadelschutzhülse 20. Gleichzeitig wird im Zusammenwirken der Schultersegmente 15 mit den beiden Vertiefungen 22 dafür gesorgt, dass die Nadelschutzhülse 20 bereits bei dem Einschieben solch eine Verdrehposition relativ zur Deckhülse 10 einnimmt, dass die Schnapper 24 mit den Anschlägen 16 fluchten. Die Schnapper 24 werden aufgrund ihres radialen Überstands im proximalen Innenmantelbereich 12 der Deckhülse 10 gegen ihre eigenen elastischen Rückstellkräfte radial einwärts gebogen. Sobald die Schnapper 24 die Anschläge 16 überschoben haben, schnappen sie aufgrund ihrer eigenen elastischen Rückstellkräfte wieder nach radial auswärts vor, so dass ihre distalen Stirnflächen dann den Anschlägen 16 unmittelbar zugewandt gegenüberliegen. In diesem Sperreingriff der Schnapper 24 ist ein Zurückschieben der Nadelschutzhülse 20 relativ zur Deckhülse 10 nicht mehr möglich. Die Schnapper 24 liegen nun vor den Anschlägen 16 in einer Verbreiterung 18 des Kanalabschnitts 17b der Deckhülse 10. Es stoßen im Sperreingriff, genauer gesagt, lediglich die links und rechts von dem abgeschrägten Mittenbereich 26 verbleibenden Randbereiche der Schnapper 24 gegen die Anschläge 16. Wird die Nadelschutzhülse 20 noch ein Stück weiter vorgeschoben, so stoßen die Schultern 23 gegen die Schultersegmente 15. Durch das Zusammenwirken der Schultersegmente 15 und der Schultern 23 soll in erster Linie verhindert werden, dass die Nadelschutzhülse 20 nach vorne aus der Deckhülse 10 herausfallen kann. Von außen sind die Schnapper 24 allenfalls noch mit einem spitzen Werkzeug von der proximalen Stirnseite der Deckhülse 10 her zugänglich.

Im nächsten Montageschritt wird das Entsicherungsmittel 30 in der in Figur 3 dargestellten Verdrehlage relativ zu den anderen Komponenten hinter die Nadelschutzhülse 20 ebenfalls in die Deckhülse 10 hinein geschoben. Die Zungen 32 werden bei dem Einschieben in den Kanälen 17 seitlich eng geführt. Das Einschieben ist beendet, wenn die Nocken 35 in den Bereich der Durchbrechungen 14 gelangen und die Zungen 32 dadurch nach außen vorschnappen können.

Anschließend werden die Betätigungselemente 36 auf die Nocken 35 aufgeklebt oder anderweitig sicher befestigt. Die Betätigungselemente 36 sind so geformt, dass sie gegen die Außenmantelfläche der Deckhülse 10 drücken und dadurch jeglichen radialen Druck bei der manuellen Betätigung von den Zungen 32 abhalten. Die Betätigungselemente 36 dienen somit gleichzeitig auch der Abstützung des Entsicherungsmittels 30 bzw. der Zungen 32 an der Deckhülse 10. Die Betätigungselemente 36 könnten auch einstückig an den Nocken 35 geformt sein, beispielsweise pilzförmig. In solcher Ausbildung würden sie bei dem Durchführen durch die Durchbrechungen 14 an den jeweiligen Nocken angelegt und würden sich nach dem Durchführen elastisch aufweiten, insbesondere abspreizen, um die Abstützung zu erhalten.

Vor dem Verbinden der Deckhülse 10 mit der Aufsteckhülse 2 wird die Druckfeder 29 (Figur 5) in die vormontierte Nadelschutzhülse 20 bis gegen den Boden der Nadelschutzhülse 20 eingeschoben.

In einem letzten Montageschritt wird die Deckhülse 10 mit den darin aufgenommenen Komponenten 20 und 30 sowie der Druckfeder 29 auf die Aufsteckhülse 2 aufgeschoben, bis der Wulst 13 in die Rille 4 eingerastet ist. Da die Deckhülse 10 im Bereich des Wulstes 13 nicht durchbrochen ist und aufgrund einer ausreichenden Steifigkeit insgesamt, ist die Rastverbindung zwischen der Rille 4 und dem Wulst 13 nicht mehr lösbar, zumindest nicht ohne Werkzeuge bzw. ohne Zerstörung.

Nimmt das Entsicherungsmittel 30 noch nicht die in Figur 5 dargestellte Rastposition ein, so kann beispielsweise im Rahmen einer Funktionsprüfung beim Hersteller, die Nadelschutzhülse 20 in ihre Distalposition zurückgeschoben werden. Die Nadelschutzhülse 20 drückt dabei das Entsicherungsmittel 30 in die distale Rastposition, wie sie das Entsicherungsmittel 30 in der Darstellung der Figur 5 einnimmt. Die Mitnahme des Entsicherungsmittels 30 erfolgt dadurch, dass die Nadelschutzhülse 20 mit einer distalen Stirnfläche 27 gegen eine ihr zugewandte Stirnfläche 33 des Entsicherungsmittels 30 drückt. Durch Eingriff der beiden Wulste 6 in die Rille 34 des Entsicherungsmittels 30 wird eine lösbare Rastverbindung hergestellt. Die Rastverbindung ist jedoch ausreichend fest, um ein Lösen aufgrund der im Gebrauch üblichen Vibrationen, Stöße und dergleichen, zu verhindern.

Die Funktion der Nadelschutzvorrichtung ist am besten aus den Figuren 5 und 6 ersichtlich.

Figur 5 zeigt die Nadelschutzvorrichtung komplett montiert und an einem nicht dargestellten Injektionsgerät befestigt. Die Aufsteckhülse 2 umgibt einen Ampullenhalter 1. Der Ampullenhalter 1 ist hülsenförmig und nimmt eine Ampulle auf, die mit einem Medikamentfluid gefüllt ist. Der Ampullenhalter 1 ist bis gegen den Ring 8 in die Aufsteckhülse 2 eingeschoben. Die Segmente 7 (Figur 3) geben die Sicht auf den Ampullenhalter 1 frei. Eine mit einem Ampullenauslass in üblicher Weise verbundene Injektionsnadel N ragt über die proximalen Enden der Aufsteckhülse 2 und der Deckhülse 10 hinaus vor. Die Nadelschutzhülse 20 nimmt in der Darstellung der Figur 5 ihre Schutzstellung ein, in der sie die Injektionsnadel N über die gesamte Länge schützend umgibt. Die Schnapper 24 liegen ihrem jeweiligen Anschlag 16 axial zugewandt gegenüber, d.h. sie stoßen an ihren jeweiligen Anschlag 16 an. Das Entsicherungsmittel 30 nimmt seine Raststellung ein, in der ein Eingriff der Zungen 32 mit den Schnappern 24 nicht besteht. Die Nadelschutzhülse 20 ist somit gegen ein Zurückschieben gesperrt.

Zwischen den Hülsensegmenten 7 und der Deckhülse 10 ist ein Ringraum 9 gebildet, in den hinein die Nadelschutzhülse 20 bis in ihre Distalposition verschoben werden kann, wenn der Sperreingriff zwischen den Schnappern 24 und den Anschlägen 16 vorher gelöst wurde. Das Lösen erfolgt durch Verschiebung des Entsicherungsmittels 30 in die proximale Richtung.

Nachfolgend wird der Funktionsablauf bei einer Injektion beschrieben, wobei stets auf alle Figuren verwiesen sei.

Nachdem eine die Injektionsnadel N noch umgebende Schutzkappe entfernt worden ist, kann eine erste Injektion vorgenommen werden.

Unmittelbar vor der Injektion schiebt der Verwender das Entsicherungsmittel 30 mittels der Betätigungselemente 36 in die proximale Richtung. Im Zuge der Verschiebung des Entsicherungsmittels 30 gleitet jede der Zungen 32 über den ihr zugeordneten Schnapper 24. Da die Schnapper 24 nach außen über die ihnen zugewandten Gleitkontaktflächen der Zungen 32 hinaus vorstehen, werden die Schnapper 24 radial einwärts gedrückt, wenn die Zungen 32 mit ihren Gleitkontaktflächen an den Schnappern 24 entlang gleiten. Das Entsicherungsmittel 30 wird bis in eine proximale Endstellung vorgeschoben. Dabei werden die Schnapper 24 von den Anschlägen 16 weggebogen, so dass die Nadelschutzhülse 20 in den Ringraum 9 hinein geschoben werden kann, wobei die Schnapper 24 elastisch gegen die Zungen 32 drücken. Die Zungen 32 sind so breit wie die verjüngten bzw. angeschrägten Mittenbereiche 26 (Figur 3) der Schnapper 24. Die Zungen 32 sind zu ihren proximalen Stirnflächen hin ebenfalls verjüngt. Indem entweder die Schnapper 24 oder die Zungen 32 oder beide verjüngt sind, wird eine Selbsthemmung, die ein Überschieben der Schnapper 24 behindern könnte, besonders sicher verhindert.

Nachdem die Sperre der Nadelschutzhülse 20 gelöst worden ist, wird das Injektionsgerät auf die Haut aufgesetzt, so dass die Nadelschutzhülse 20 mit ihrem Boden auf der Haut aufliegt und die Injektionsnadel N in etwa senkrecht zur Hautoberfläche weist. In dieser Injektionsstellung wird die Nadelschutzhülse 20 durch Druck des Injektionsgeräts gegen die Hautoberfläche in den Ringraum 9 bis in eine Distalstellung hinein verschoben. Gleichzeitig durchsticht die Injektionsnadel N die Haut und dringt in das darunter liegende Gewebe ein.

Während der Verschiebung in den Ringraum 9 hinein, gelangt eine distale Stirnfläche 27 (Figur 2) der Nadelschutzhülse 20 in Eingriff mit der Stirnfläche 33 des Entsicherungsmittels 30. Der Eingriff besteht in einem losen Aneinanderstoßen der beiden Stirnflächen 27 und 33. Auf diese Weise drückt die Nadelschutzhülse 20 das Entsicherungsmittel 30 bis in die in Figur 5 dargestellte Raststellung, in welcher die beiden Wulste 6 in die Vertiefung 34 eingreifen. Wird nach der Injektion die Injektionsnadel wieder aus dem Gewebe herausgezogen und die Nadelschutzvorrichtung entlastet, so schiebt die Nadelschutzhülse 20 unter der Kraft der Druckfeder 29 wieder in die proximale Richtung. Das Entsicherungsmittel 30 verbleibt bei dem Verschieben der Nadelschutzhülse 20 in seiner Raststellung. Sobald die Nadelschutzhülse 20 soweit vorgeschoben worden ist, dass die Schnapper 24 vor die Anschläge 16 gelangt sind, schnappen die Schnapper 24 aufgrund der durch das Abbiegen in ihnen erzeugten Rückstellkräfte wieder radial auswärts, so dass sie den Anschlägen 16 wieder gegenüberliegen und der Sperreingriff wieder hergestellt ist.

Das Injektionsgerät kann mit der in gesperrter Schutzstellung befindlichen Nadelschutzhülse entsorgt werden. Ebenso erfüllt die Nadelschutzvorrichtung ihre Schutzfunktion auch bei mehrfachen Injektionen. Und schließlich kann die Nadelschutzvorrichtung auch nacheinander in Verbindung mit mehreren Injektionsgeräten verwendet werden. Grundsätzlich kann sie sogar in der Ausbildung als Aufsteckvorrichtung mit unterschiedlichen Injektionsgeräten verwendet werden, wenn nämlich die Aufsteckhülse 2 an das jeweilige Injektionsgerät angepasst geformt ist. Bei einem Großteil der Injektionsgeräte kann angenommen werden, dass die Ampullen in etwa gleiche Querschnitte aufweisen, so dass tatsächlich nur Anpassungen der Aufsteckhülse 2 erforderlich sind.

### Bezugszeichenliste

- 1: Ampullenhalter
- 2: Basiskörper, Aufsteckhülse
- 3: distaler Hülsenbereich
- 4: Rille
- 5: Schulter
- 6: Wulst
- 7: innerer Hülsenbereich, Hülsensegmente
- 8: innerer Hülsenbereich, Ring
- 9: Ringraum
- 10: Basiskörper, Deckhülse
- 11: distaler Hülsenbereich
- 12: proximaler Hülsenbereich
- 13: Wulst
- 14: Durchbrechung
- 15: Schultersegment
- 16: Sperrmittel, Schnapperanschlag
- 17: Kanal
- 17a: proximaler Kanalabschnitt
- 17b: distaler Kanalabschnitt
- 18: Verbreiterung
- 19: -
- 20: Nadelschutzhülse
- 21: Öffnung
- 22: Vertiefung
- 23: Schulter
- 24: Sperrmittel, Schnapper
- 25: Ausschnitt
- 26: verjüngter Mittenbereich
- 27: Stirnfläche
- 28: -
- 29: Rückstellelement
- 30: Entsicherungsmittel
- 31: Ring
- 32: Zunge
- 33: Stirnfläche
- 34: Vertiefung
- 35: Nocken
- 36: Betätigungselement

## Patentansprüche

1. Nadelschutzvorrichtung für ein Injektionsgerät, die Nadelschutzvorrichtung umfassend:
a) einen hülsenförmigen Basiskörper (2, 10), der mit einem Gehäuse des Injektionsgeräts verschiebesicher verbindbar ist oder durch ein Gehäuse des Injektionsgeräts gebildet wird,
b) eine Nadelschutzhülse (20), die an dem Basiskörper (2, 10) axial verschiebbar gelagert ist,
c) wobei die Nadelschutzhülse (20) in einer proximalen Schutzstellung eine Injektionsnadel (N) des Injektionsgeräts umgibt und in einer Distalstellung die Injektionsnadel (N) freigibt,
d) ein erstes Sperrmittel (24) und ein zweites Sperrmittel (16), von denen das eine verschiebesicher mit der Nadelschutihülse (20) und das andere verschiebesicher mit dem Basiskörper (2, 10) verbunden ist,
e) wobei in der Schutzstellung der Nadelschutzhülse (20) ein Sperreingriff **dadurch** gebildet wird, dass die Sperrmittel (24, 16) axial gegeneinander stossen und **dadurch** eine Verschiebung der Nadelschutzhülse (20) in die Distalposition verhindern,
f) und ein Entsicherungsmittel (30)zum Lösen des Sperreingriffs,
**dadurch gekennzeichnet, dass**
g) das erste Sperrmittel (24) durch einen elastisch biegbaren Schnapper (24) gebildet wird, der mit der Nadelschutzhülse (20) oder dem Basiskörper (2, 10) steif verbunden ist,
h) und der Sperreingriff der Sperrmittel (24, 16) durch Biegen des Schnappers (24) gegen eine durch das Biegen in dem Schnapper (24) erzeugte Rückstellkraft gelöst wird.

2. Nadelschutzvorrichtung nach Anspruch 1, **dadurch gekennzeichnet, dass** das Entsicherungsmittel (30) an dem Basiskörper (2, 10) relativ zu der Nadelschutzhülse (20) oder an der Nadelschutzhülse (20) relativ zu dem Basiskörper (2, 10) bewegbar gelagert ist.

3. Nadelschutzvorrichtung nach einem der vorhergehenden Ansprüche, **dadurch gekennzeichnet, dass** das Entsicherungsmittel (30) an dem Basiskörper (2, 10) relativ zu der Nadelschutzhülse (20) verschiebbar gelagert ist, derart, dass es bei einem Verschieben an dem ersten Sperrmittel (24) entlang gleitet und das erste Sperrmittel (24) aus dem Sperreingriff biegt.

4. Nadelschutzvorrichtung nach einem der vorhergehenden Ansprüche, **dadurch gekennzeichnet, dass** in der Schutzstellung der Nadelschutzhülse (20) zwischen dem Entsicherungsmittel (30) und dem Basiskörper (2, 10) ein Rasteingriff besteht, durch den das Entsicherungsmittel (30) gegen eine Mitnahme durch die sich in die Schutzstellung bewegende Nadelschutzhülse (20) gesichert ist.

5. Nadelschutzvorrichtung nach einem der vorhergehenden Ansprüche, **dadurch gekennzeichnet, dass** die Nadelschutzhülse (20) bei dem Verschieben in die Distalstellung das Entsicherungsmittel (30) mitnimmt, bis das Entsicherungsmittel (30) in einen Rasteingriff mit dem Basiskörper (2, 10) gelangt.

6. Nadelschutzvorrichtung nach einem der zwei vorhergehenden Ansprüche, **dadurch gekennzeichnet, dass** der Rasteingriff des Entsicherungsmittels (30) lösbar ist.

7. Nadelschutzvorrichtung nach einem der vorhergehenden Ansprüche, **dadurch gekennzeichnet, dass** die Nadelschutzhülse (20) bei dem Verschieben in die Distalstellung lose gegen das Entsicherungsmittel (30) drückt und das Entsicherungsmittel (30) mit verschiebt.

8. Nadelschutzvorrichtung nach einem der vorhergehenden Ansprüche, **dadurch gekennzeichnet, dass** das erste Sperrmittel (24) axial von der Nadelschutzhülse (20) oder dem Basiskörper (2, 10) abragt, radial biegbar ist und in der Schutzstellung der Nadelschutzhülse (20) gegen seine elastische Rückstellkraft aus dem Sperreingriff gebogen wird.

9. Nadelschutzvorrichtung nach einem der vorhergehenden Ansprüche, **dadurch gekennzeichnet, dass** das zweite Sperrmittel (16) an einer Mantelfläche des Basiskörpers (2, 10) oder der Nadelschutzhülse (20) gebildet ist.

10. Nadelschutzvorrichtung nach einem der vorhergehenden Ansprüche, **dadurch gekennzeichnet, dass** das Entsicherungsmittel (30) das erste Sperrmittel (24) zum Lösen des Sperreingriffs überschiebt und radial einwärts biegt.

11. Nadelschutzvorrichtung nach einem der vorhergehenden Ansprüche, **dadurch gekennzeichnet, dass**
- der Basiskörper (2, 10) eine Deckhülse (10) aufweist, in die hinein die Nadelschutzhülse (20) verschiebbar ist,
- die Deckhülse (10) eine Durchbrechung (14) aufweist,
- und ein Nocken (35) des Entsicherungsmittels (30) durch die Durchbrechung (14) ragt.

12. Nadelschutzvorrichtung nach dem vorhergehenden Anspruch, **dadurch gekennzeichnet, dass** der Nocken (35) an einer Außenmantelfläche der Deckhülse (10) gegen eine radial einwärts gerichtete Bewegung abgestützt ist.

13. Nadelschutzvorrichtung nach einem der vorhergehenden Ansprüche, **dadurch gekennzeichnet, dass**
- der Basiskörper (2, 10) einen inneren Hülsenbereich (7, 8) und eine Deckhülse (10) aufweist, welche den inneren Hülsenbereich (7, 8) umgibt,
- zwischen dem inneren Hülsenbereich (7, 8) und der Deckhülse (10) ein Ringraum (9) verbleibt,
- und die Nadelschutzhülse (20) in den Ringraum (9) hinein verschiebbar ist.

14. Nadelschutzvorrichtung nach einem der vorhergehenden Ansprüche, **dadurch gekennzeichnet, dass** an dem Basiskörper (2, 10) und der Nadelschutzhülse (20) ein Rückstellelement (29) abgestützt ist, das bei der Verschiebung der Nadelschutzhülse (20) in die Distalstellung gespannt wird und die Nadelschutzhülse (20) aus der Distalstellung in die Schutzstellung vorschiebt.

15. Nadelschutzvorrichtung nach einem der vorhergehenden Ansprüche, **dadurch gekennzeichnet, dass** der Basiskörper (2, 10) auf ein Gehäuse eines Injektionsgeräts aufsteckbar ist.

## Claims

1. A needle protection device for an injection instrument, the needle protection device including:
a) a sleeve-shaped base body (2, 10) which can be connected non-slidably to a casing of the injection instrument or is formed by a casing of the injection instrument,
b) a needle protection sleeve (20) which is axially slidably mounted on the base body (2, 10),
c) wherein the needle protection sleeve (20) in a proximal protection position surrounds an injection needle (N) of the injection instrument and in a distal position exposes the injection needle (N),
d) a first locking means (24) and a second locking means (16) of which one is non-slidably connected to the needle protection sleeve (20) and the other is non-slidably connected to the base body (2, 10),
e) wherein in the protection position of the needle protection sleeve (20) a locking engagement is formed by the locking means (24, 16) bearing axially against each other and thereby preventing sliding displacement of the needle protection sleeve (20) into the distal position, and
f) a release means (30) for releasing the locking engagement,
**characterised in that**
g) the first locking means (24) is formed by an elastically bendable latch (24) which is rigidly connected to the needle protection sleeve (20) or the base body (2, 10), and
h) the locking engagement of the locking means (24, 16) is released by bending the latch (24) against a return force produced by the bending movement in the latch (24).

2. A needle protection device according to claim1 **characterised in that** the release means (30) is mounted on the base body (2, 10) movably relative to the needle protection sleeve (20) or on the needle protection sleeve (20) relative to the base body (2, 10).

3. A needle protection device according to one of the preceding claims **characterised in that** the release means (30) is mounted on the base body (2, 10) slidably relative to the needle protection sleeve (20) in such a way that in a sliding movement it slides along the first locking means (24) and bends the first locking means (24) out of the locking engagement.

4. A needle protection device according to one of the preceding claims **characterised in that** a latching engagement obtains in the protection position of the needle protection sleeve (20) between the release means (30) and the base body (2, 10), by which latching engagement the release means (30) is secured against entrainment by the needle protection sleeve (20) as it moves into the protection position.

5. A needle protection device according to one of the preceding claims **characterised in that** the needle protection sleeve (20) entrains the release means (30) in the sliding movement into the distal position until the release means (30) comes into latching engagement with the base body (2, 10).

6. A needle protection device according to one of the two preceding claims **characterised in that** the latching engagement of the release means (30) is releasable.

7. A needle protection device according to one of the preceding claims **characterised in that** the needle protection sleeve (20) presses loosely against the release means (30) in the sliding movement into the distal position and displaces the release means (30) therewith.

8. A needle protection device according to one of the preceding claims **characterised in that** the first locking means (24) projects axially from the needle protection sleeve (20) or the base body (2, 10), is radially bendable and in the protection position of the needle protection sleeve (20) is bent out of the locking engagement against its elastic return force.

9. A needle protection device according to one of the preceding claims **characterised in that** the second locking means (16) is formed on a peripheral surface of the base body (2, 10) or the needle protection sleeve (20).

10. A needle protection device according to one of the preceding claims **characterised in that** the release means (30) pushes over the first locking means (24) to release the locking engagement and bends it radially inwards.

11. A needle protection device according to one of the preceding claims **characterised in that**
- the base body (2, 10) has a cover sleeve (10) into which the needle protection sleeve (20) is displaceable,
- the cover sleeve (10) has a through opening (14), and
- a projection (35) of the release means (30) projects through the through opening (14).

12. A needle protection device according to the preceding claim **characterised in that** the projection (35) is supported at an outside peripheral surface of the cover sleeve (10) against a radially inwardly directed movement.

13. A needle protection device according to one of the preceding claims **characterised in that**
- the base body (2, 10) has an inner sleeve region (7, 8) and a cover sleeve (10) which surrounds the inner sleeve region (7, 8),
- an annular space (9) remains between the inner sleeve region (7, 8) and the cover sleeve (10), and
- the needle protection sleeve (20) is slidable into the annular space (9).

14. A needle protection device according to one of the preceding claims **characterised in that** a return element (29) is supported against the base body (2, 10) and the needle protection sleeve (20), which return element is stressed upon the sliding movement of the needle protection sleeve (20) into the distal position and advances the needle protection sleeve (20) out of the distal position into the protection position.

15. A needle protection device according to one of the preceding claims **characterised in that** the base body (2, 10) can be fitted on to a casing of an injection instrument.

## Revendications

1. Dispositif de protection d'aiguille pour appareil d'injection, le dispositif de protection d'aiguille comportant :
a) un corps de base en forme de manchon (2, 10), qui peut être relié de façon stationnaire à un boîtier de l'appareil d'injection ou est formé par un boîtier de l'appareil d'injection,
b) un manchon de protection d'aiguille (20), qui est logé sur le corps de base (2, 10) de manière à pouvoir se déplacer dans le sens axial,
c) le manchon de protection d'aiguille (20), dans une position de protection proximale, entourant une aiguille d'injection (N) de l'appareil d'injection et dans une position distale, libérant l'aiguille d'injection (N),
d) un premier moyen de blocage (24) et un second moyen de blocage (16), parmi lesquels l'un est relié de façon stationnaire au manchon de protection d'aiguille (20) et l'autre est relié de façon stationnaire au corps de base (2, 10),
e) sachant que dans la position de protection du manchon de protection d'aiguille (20), un engagement de blocage est formé en ce sens que le moyen de blocage (24, 16) sont en butée l'un contre l'autre dans le sens axial et empêchent de ce fait un déplacement du manchon de protection d'aiguille (20) dans la position distale,
f) et un moyen de déblocage (30) destiné à libérer l'engagement de blocage,
**caractérisé en ce que**
g) le premier moyen de blocage (24) est formé par un loquet (24) pouvant fléchir de façon élastique, qui est relié de façon rigide au manchon de protection d'aiguille (20) ou au corps de base (2, 10),
h) et l'engagement de blocage des moyens de blocage (24, 16) est libéré en fléchissant le loquet (24) dans le sens inverse d'une force de rappel générée par le fléchissement dans le loquet (24).

2. Dispositif de protection d'aiguille selon la revendication 1, **caractérisé en ce que** le moyen de déblocage (30) est logé sur le corps de base (2, 10) de manière à pouvoir se déplacer par rapport au manchon de protection d'aiguille (20) ou sur le manchon de protection d'aiguille (20) de manière à pouvoir se déplacer par rapport au corps de base (2, 10).

3. Dispositif de protection d'aiguille selon l'une quelconque des revendications précédentes, **caractérisé en ce que** le moyen de déblocage (30) est logé sur le corps de base (2, 10) de manière à pouvoir se déplacer par rapport au manchon de protection d'aiguille (20), de telle sorte qu'il glisse le long du premier moyen de blocage (24) à l'occasion d'un déplacement et que le premier moyen de blocage (24) sort de l'engagement de blocage en fléchissant.

4. Dispositif de protection d'aiguille selon l'une quelconque des revendications précédentes, **caractérisé en ce qu'**un engagement d'encliquetage, empêchant le moyen de déblocage (30) d'être entraîné par le manchon de protection d'aiguille (20) se déplaçant dans la position de protection, est présent dans la position de protection du manchon de protection d'aiguille (20) entre le moyen de déblocage (30) et le corps de base (2, 10).

5. Dispositif de protection d'aiguille selon l'une quelconque des revendications précédentes, **caractérisé en ce que** le manchon de protection d'aiguille (20), lorsqu'il se déplace dans la position distale, entraîne le moyen de déblocage (30) jusqu'à ce que le moyen de déblocage (30) parvient en engagement d'encliquetage avec le corps de base (2, 10).

6. Dispositif de protection d'aiguille selon l'une quelconque des deux revendications précédentes, **caractérisé en ce que** l'engagement d'encliquetage du moyen de déblocage (30) peut être libéré.

7. Dispositif de protection d'aiguille selon l'une quelconque des revendications précédentes, **caractérisé en ce que** le manchon de protection d'aiguille (20), lorsqu'il se déplace dans la position distale, effectue une pression lâche contre le moyen de déblocage (30) et déplace le moyen de déblocage (30) par entraînement.

8. Dispositif de protection d'aiguille selon l'une quelconque des revendications précédentes, **caractérisé en ce que** le premier moyen de blocage (24) dépasse dans le sens axial du manchon de protection d'aiguille (20) ou du corps de base (2, 10), peut être fléchi dans le sens radial et, dans la position de protection du manchon de protection d'aiguille (20), sort en fléchissant de l'engagement de blocage dans le sens inverse de sa position de rappel élastique.

9. Dispositif de protection d'aiguille selon l'une quelconque des revendications précédentes, **caractérisé en ce que** le second moyen de blocage (16) est formé sur une surface de revêtement du corps de base (2, 10) ou du manchon de protection d'aiguille (20).

10. Dispositif de protection d'aiguille selon l'une quelconque des revendications précédentes, **caractérisé en ce que** le moyen de déblocage (30) fait glisser le premier moyen de blocage (24) pour libérer l'engagement de blocage et le fléchit vers l'intérieur dans le sens radial.

11. Dispositif de protection d'aiguille selon l'une quelconque des revendications précédentes, **caractérisé en ce que**
- le corps de base (2, 10) comprend un manchon de couverture (10) à l'intérieur duquel le manchon de protection d'aiguille (20) peut se déplacer,
- le manchon de couverture (10) comprend une perforation (14),
- et une came (35) du moyen de déblocage (30) dépasse à travers la perforation (14).

12. Dispositif de protection d'aiguille selon l'une quelconque des revendications précédentes, **caractérisé en ce que** la came (35) s'appuie contre une surface de revêtement externe du manchon de couverture (10) dans le sens inverse d'un déplacement orienté radialement vers l'intérieur.

13. Dispositif de protection d'aiguille selon l'une quelconque des revendications précédentes, **caractérisé en ce que**
- le corps de base (2, 10) comprend une zone interne de manchon (7, 8) et un manchon de couverture (10), lequel entoure la zone interne de manchon (7, 8),
- il reste un espace annulaire (9) entre la zone interne de manchon (7, 8) et le manchon de couverture (10),
- et le manchon de protection d'aiguille (20) peut se déplacer à l'intérieur de l'espace annulaire (9).

14. Dispositif de protection d'aiguille selon l'une quelconque des revendications précédentes, **caractérisé en ce qu'**un élément de rappel (29), qui est tendu lorsque le manchon de protection d'aiguille (20) se déplace dans la position distale et avance le manchon de protection d'aiguille (20) de la position distale à la position de protection, s'appuie contre le corps de base (2, 10) et le manchon de protection d'aiguille (20).

15. Dispositif de protection d'aiguille selon l'une quelconque des revendications précédentes, **caractérisé en ce que** le corps de base (2, 10) peut être monté sur un boîtier d'un appareil d'injection.
